# EUROPEAN PATENT APPLICATION

(11) **EP 4 201 313 A1**
(43) Date of publication of application: **28.06.2023**
(21) Application number: 23152780.5
(22) Date of filing: 10.04.2019
(51) Int. Cl.: A61B 5/00, A61B 5/01, A61B 5/08

(54) **SYSTEM AND METHOD FOR MONITORING PHYSIOLOGICAL PARAMETERS, IN PARTICULAR OF A NEWBORN, AND RELATIVE SENSORIZED TEXTILE ITEM FOR THE DETECTION AND TRANSMISSION OF PHYSIOLOGICAL PARAMETERS**

(30) Priority: 24.04.2018 IT 201800004810
(62) Divisional of application: 19722699.6
(71) Applicant: Comftech S.r.L., 20900 Monza (MB) (IT)
(72) Inventor: MOLTANI, Lara Alessia Laura, 20900 Monza (IT); ORLANDI, Luca Domenico, 20900 Monza (IT)
(74) Representative: Perani & Partners S.p.A.

(57) **Abstract**

Sensorized textile item (1) for the detection and transmission of physiological parameters, including a textile support (2), made of elastic material and configured to be worn by a newborn (U); said support (2) comprising a band (3) having a rectangular peripheral development and defining respective opposite ends (3a, 3b); at least one sensor (8) arranged at an inner surface (2a) of the textile support (2) to be in contact with the newborn (U), said sensor (8) being configured to detect a plurality of signals representative of respective physiological parameters of the newborn (U); and a wireless transceiver (9) configured to receive said signals from the sensor (8) and forward them to a user interface device (10); a reversible hooking member (11) of said transceiver (9) to an outer surface (2b) of said textile support (2) opposite said inner surface (2a); said hooking member (11) presenting a first and a second element (11a, 11b) engageable/disengageable from each other and respectively associated to the textile support (2) and to the transceiver (9); said support (2) further comprising resolvable engagement means (4) arranged at said ends (3a, 3b) to close the band (3) in a ring around the newborn (U), the sensor (8) being arranged proximate to a first end (3a) of said ends (3a, 3b) of the band (3), the first and the second element (11a, 11b) of the reversible hooking member (11) being arranged at a second end (3b) of said ends (3a, 3b) of the band (3), for arranging said at least one sensor (8) at the chest of the newborn (U), and said reversible hooking member (11) at the back of the newborn (U), and said resolvable engagement means (4) at the side of the newborn (U).

## Description

The present invention relates to a system and a method for monitoring the physiological parameters, in particular of a newborn, to allow a user, typically hospital nursing staff, to monitor the position and the health status of the newborn.

The present invention also relates to a sensorized textile item used in the above-mentioned system, for the detection and transmission of physiological parameters.

In greater detail, the present invention relates to a textile item and/or a garment in particular for infants, capable of detecting and measuring the physiological parameters of the newborn and to transmit it to a user interface.

As is well known, there are clothing items which are internally provided with sensors for the detection of some physiological parameters such as for example the heart beat (by ECG). These sensors are able to send electromagnetic signals to a user interface of the mobile type, typically a "tablet" or a "smartphone".

These items are mostly used in the context of sports, to provide the user with information in real time about the physiological conditions during training. In this context, the clothing items are constituted by sport shirts inside which sensors and the suitable circuitry for the transmission of the electrical signal are integrated.

Another area of particular interest within the scope of the present invention is that of sensorized garments for medical use, for constant monitoring of the health status of users affected by particular pathologies, or for verification of the health in the first days of life.

Particularly in the latter case, and especially for babies prematurely born, a constant control of some vital parameters of the infant becomes necessary in order to possibly intervene in emergency cases.

For this reason, sensorized clothing items for infants constituted by suits, shirts or bodysuits are provided, implementing the constant monitoring of the newborn in a non-invasive manner and without the use of external electrodes.

The sensorized clothing items for infants in fact have one or more sensors positioned at a front zone of the back and then next to the heart and lungs to detect more efficiently at least the heartbeat and respiratory motion of the newborn.

The sensors, conveniently made of pliable material to provide the maximum comfort to the newborn child, are integrated to the textile item (garment) and connected by conductive yarns to transmission devices of the electric signal.

In particular, the conductive yarns run up to a transmitter which is also integrated into the garment and localized in a region, for example one side) that does not cause any disturbance in the movements of the infant and during service operations to it.

The transmitter will then send the signals representative of the physiological parameters of the newborn to a user interface that can be managed within the home environment directly by the parents or in a hospital by the nursing staff.

The clothing items described above, and in particular those used for the infants, although able to monitor the health status of the newborn in an efficient manner, however, has some major applicative drawbacks and limitations.

First of all, a first drawback of the above described prior art is determined by the presence of the whole electronic circuitry integrated to the fabric that connects the sensor to the user interface device.

In fact, it should be noted that the circuitry, and in particular the transmitter, even if incorporated in the fabric are still particularly uncomfortable when worn since it is not elastic and not able to optimally adapt to the profile of the user.

Furthermore, the transmitter, typically of large dimensions and made of rigid materials (plastic), even if located in an area of less disturbance to the newborn, provides however a considerable inconvenience for the newborn, in particular if the respective garment is adherent to the body.

In this case, in fact, the transmitter is compressed by the elastic structure of the fabric against the body of the infant with the consequent damage to the superficial tissues of the skin of the newborn.

It should also be noted that the aforementioned electronic circuitries, constituted as specified above by the transmitter and by the respective integrated electrical connections to the fabric, are not breathable and therefore can generate damage to the notoriously very delicate skin of the newborn in the first days after birth.

The above-mentioned drawbacks are even more felt precisely in the medical field, as the sensorized clothing item must be kept worn by the user for a prolonged time. In the specific neonatal medical sector in which the present invention is particularly inserted, it is in fact necessary to maintain the sensorized clothing item worn by the newborn for the whole time of admission.

In this context, a further important drawback derives precisely from the need to change frequently the sensorized clothing item to implement care measures for the newborn, to allow recharging of the supply batteries (or in general the maintenance of the components), to always maintain optimal the garment's hygienic conditions, and to prevent any physiological liquids of the newborn from interfering with the correct functioning of the sensors and electronic components.

Therefore, the frequent replacement of the clothing item is particularly awkward for the medical staff and being particularly frequent is also disadvantageous in terms of overall time and cost of use.

Another important drawback of sensorized clothing items of the known type is given by the poor versatility of the clothing itself that must be provided in different sizes to be used in an optimum manner.

It should be noted that the accuracy of the sensors is directly proportional to the correct position of the same with respect to the body of the newborn. In this context, the use of a garment of a size not suitable for the user involves the non-optimal positioning of the sensors with consequent errors in the detection of physiological parameters.

Furthermore, as described above, a garment that is too tight is not very comfortable due to the presence of rigid components, while at the same time a garment too large could determine the detachment of the sensor from the user's skin with the consequent interruption of the monitoring of physiological parameters.

In this situation, especially in hospitals, it is proved to be necessary to have a series of clothing items of different sizes with the consequent disadvantages in terms of costs and overall dimensions.

Finally, another important drawback of sensorized clothing items is the structural complexity of the entire item, to which the sensors and the aforementioned electronic components are integrated.

In fact, to be able to precisely position the sensors, the entire clothing item is made with particular care and with minimum tolerances.

In this context, the technical task underlying the present invention is to propose a sensorized textile item for the detection and transmission of physiological parameters, as well as a clothing item and a system for monitoring the physiological parameters, in particular of an infant, which overcome the drawbacks of the above-mentioned prior art.

In particular, it is an object of the present invention to provide a sensorized textile item which is particularly comfortable in that it has no components that make the use of the item inconvenient.

Another object of the present invention is to provide a comfortable sensorized textile item, which is also easy to apply to newborns, and which is free of elements that can potentially damage the tissues of the infant.

A further object of the present invention is to propose a clothing item that is versatile in that it is easily adaptable to the size of the user and at the same time capable of always precisely positioning the sensors for the detection of physiological parameters.

Another object of the invention is to provide a sensorized textile item which does not require frequent replacements deriving from operations of electrical recharging or maintenance, and in any case capable of maintaining the user's optimal hygienic conditions.

Another object of the present invention is to provide a sensorized textile item with particularly low manufacturing costs since it is structurally simple and can be integrated with any clothing item.

Finally, an object of the invention is to provide a system and a method for monitoring the physiological parameters in particular of a newborn, suitable to detect the position and health status of the newborn within a predefined area such as for example a hospital ward.

The specified technical task and the specified object are substantially achieved by a sensorized textile item for the detection and transmission of physiological parameters, a clothing item, a system and a method for monitoring the physiological parameters in particular of a newborn, comprising the technical characteristics set forth in one or more of the appended claims.

Further characteristics and advantages of the present invention will become clearer from the indicative description, and therefore non-limiting, of a preferred, but not exclusive, embodiment of a sensorized textile item for the detection and transmission of physiological parameters, as well as a clothing item and a system for monitoring the physiological parameters of a newborn, as illustrated in the appended drawings, wherein:
- Figure 1 shows a rear perspective view of a sensorized textile item according to the present invention and in a respective condition of use for detecting physiological parameters of an infant;
- Figure 2 shows a front perspective view of a sensorized textile item according to the present invention and in a respective condition of use for the detection of physiological parameters of an infant;
- Figure 3 shows a perspective and partly exploded view of the item of Figure 1 not associated with a user;
- Figure 4 shows a perspective view of the item in Figure 2 not associated to a user;
- Figures 5a and 5c respectively show top and bottom plan views of the sensorized textile item in accordance with the present invention and in a respective condition of non-use;
- Figure 5b shows an elevational side view of the sensorized textile item of Figures 5a and 5b.
- Figure 6 shows a plan view of an electronic component of the sensorized textile item;
- Figure 7 shows a front view of the clothing item for infants, according to the present invention and provided with a respective sensorized textile item; and
- Figure 8 shows a schematic view of a system for monitoring the physiological parameters of a newborn within a predefined area such as a hospital department.

With reference to the attached figures, reference number 1 globally indicates a sensorized textile item for the detection and transmission of physiological parameters.

It should be specified that the present invention finds particular and advantageous use in the neonatal field, for the detection and monitoring of the health status of a newborn in the first hours of life and within a hospital.

However, this application area should not be understood in a limiting way. In fact, the present invention is used in other areas, such as for example in a domestic or sports environment, and for any user who needs a precise and constant control of the respective physiological parameters.

Again, it should be specified that with the term "physiological parameters" used in the following discussion, some measurable parameters, typically identifying the health status of the user, are understood in a non-limiting manner.

These parameters can for example be the heartbeat and the cardiac frequency, temperature, arterial pressure, respiratory movements and frequency, etc.

With reference to Figure from 1 to 5c, the item 1 comprises a textile support 2, made of elastic material and configured to be worn by a user "U".

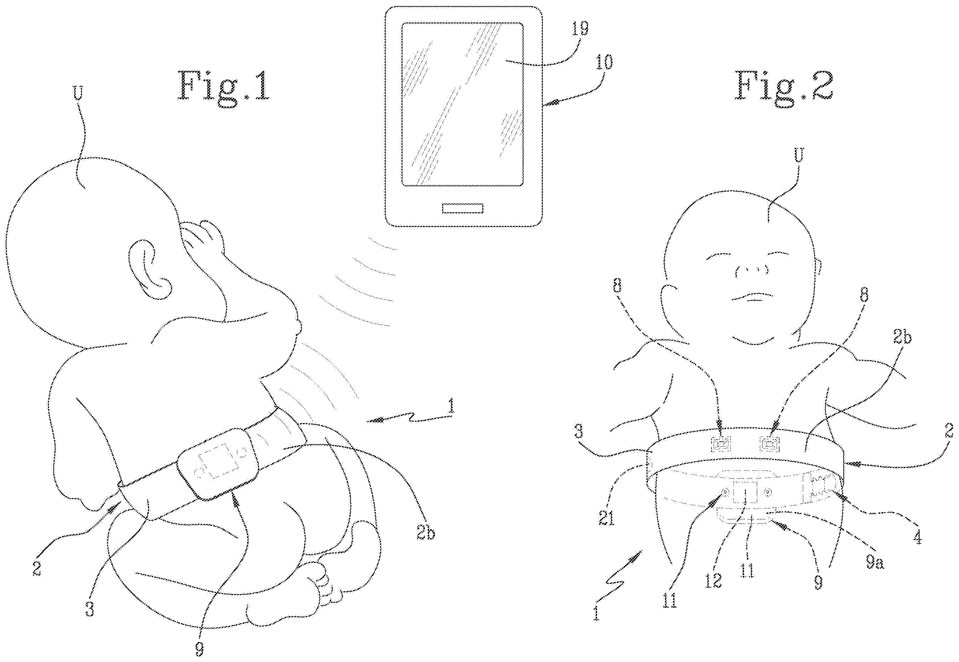

In particular, the textile support 2, is designed to wrap around and adhere to the skin of the user "U" as is shown in Figures 1 and 2.

In greater detail, the support 2 comprises a band 3, advantageously made of elastic and breathable fibre, presents a rectangular and substantially elongated peripheral development, in which are defined respective opposite ends 3a, 3b.

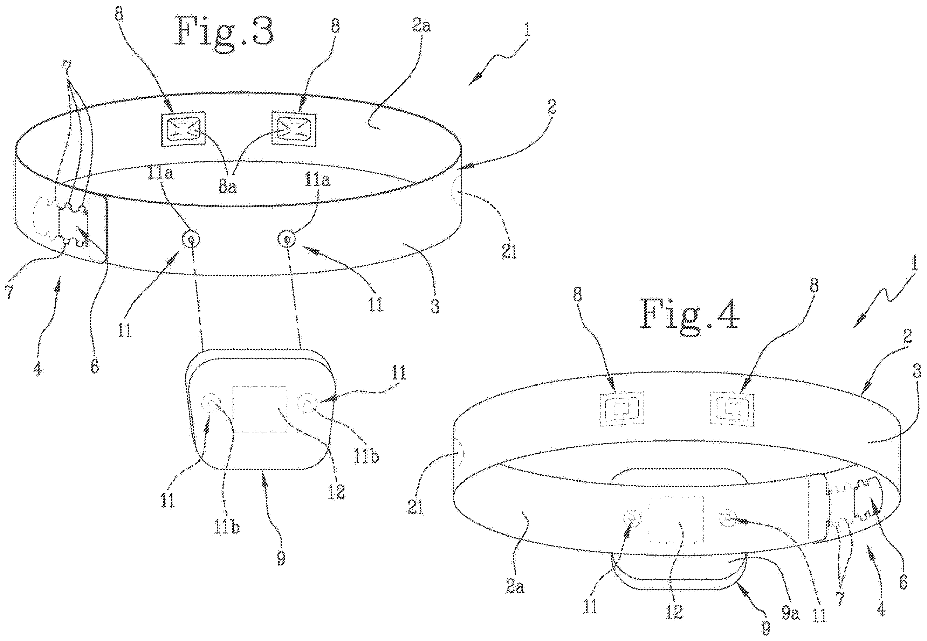

The band 3 comprises respective resolvable engagement means 4 arranged at said ends 3a, 3b and adapted to close in a ring the band 3 as is better illustrated in figures 3 and 4.

Preferably, the resolvable engagement means 4 are constituted by at least one through opening 5 formed in a first end 3a of the band 3, and at least one strap 6 integrally formed with the band 3 and projecting from a second end 3b.

The strap 6 is constituted by a portion of lesser amplitude with respect to the band 3 and extends from the respective second ends 3b along the same longitudinal development axis of the band 3.

As described above the strap 6 is made in one piece and with the same textile material of the band 3. Therefore, even the strap 6 has the same characteristics of elasticity and breathability of the band 3.

Furthermore, the strap 6 has a series of lateral projections 7 spaced apart from each other and extending along the longitudinal edges of the strap 6 itself.

Preferably, the first end 3a of the band 3 presents two through openings 5 in the form of slits, spaced from one another and having different amplitude. In fact, it should be noted that the opening 5 that is closer to the end 3a has an amplitude greater than the opening 5 that is closer to a centre line area of the band 3.

In this way, the strap 6 is insertable into the through openings 5 for mutually engaging in a reversible manner the ends 3a, 3b defining the ring configuration illustrated in Figures from 1 to 4.

In greater detail, in the closed ring configuration, the opening 5 of lesser amplitude remains engaged by mechanical interference between two adjacent lateral projections 7. It should be noted, in fact, that the dimension between two projections 7 on the opposite sides is greater than the width of the opening 5 with a smaller width just to define this mechanical constraint.

By virtue of the elastic and flexible nature with which the strap 6 is made, the projections 7 are folded and forced to pass inside the openings 5 following a manual action of moving the two ends 3a, 3b of the band away from each other.

Advantageously, by acting manually on the ends 3a, 3b of the band 3, the support 2 is easily wrapped around the torso of the user.

It should also be noted that the presence of a series of protrusions 7 brought close together and suitably spaced, allows to position the strap 6 with respect to the openings 5 by adjusting the circumference of the annular path defined by the support 2 closed around the torso in function of the size of the user "U".

The operations of removal of the support 2 from the torso of the user are also particularly simple, since it is only needed to manually move away the two ends 3a, 3b of the band 3 by forcing the projections 7 to pass through the openings 5.

In use, the aforesaid resolvable engagement means 4 are preferably positioned at the side of the user (Figure 2) to implement greater comfort and to avoid being unintentionally tampered with.

The item 1 further comprises at least one sensor 8 arranged in correspondence with an inner surface 2a of the textile support 2 adapted to be facing and in contact with the user "U".

The sensor 8 is configured to detect a plurality of signals representing respective physiological parameters of the user "U".

Preferably, as is illustrated in the accompanying drawings, there are two sensors 8, suitably spaced apart and on the band 3 in proximity of the first end 3a.

Also in this case, the number of sensors 8 provided can be any, in function of the various requirements of application. In accordance with the present invention and according to a preferred embodiment and exemplary anyway, are provided two sensors 8.

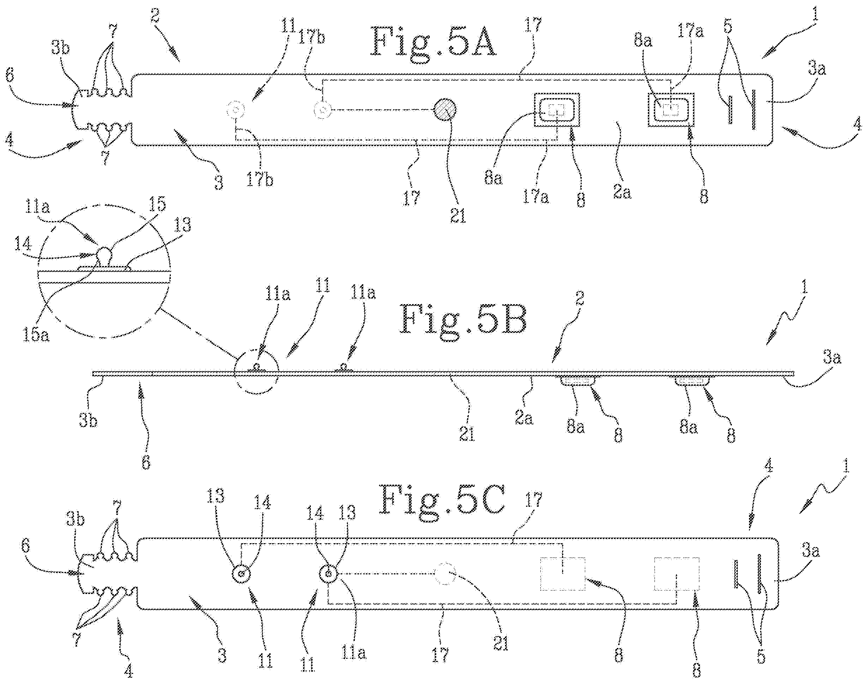

With particular reference to Figure 5b, it should be noted that each sensor 8 has a contact surface 8a with the skin of the user "U" that emerges from the inner surface 2a and which protrudes from the band 3. Advantageously, the protruding configuration of the contact surface 8a, together with the action of elastic adherence of the band 3, allows the sensor 8 to be always kept adherent to the user's skin and pressed on it to ensure the correct detection of the physiological parameter.

Preferably, the sensors 8 are of the type having an internal padding, made of a material that is soft and pliable so as to confer greater comfort for the user "U", and adapted to place the electronic components for detecting always next to and in contact with the user "U".

In accordance with a preferential embodiment of the present invention, each sensor 8 is of the type described in the patent publication PCT/IB2014/063109 owned by the Applicant.

This sensor 8 is not described in detail since it has already been extensively illustrated in the aforementioned publication.

Advantageously, in the condition of use of the ring-shaped support 2 (Figure 2), the sensors 8 are configured to be positioned at the chest of the user "U" and to detect the aforementioned physiological parameters in an optimal manner.

In addition, a temperature sensor 21, positionable in a hole formed on the inner surface 2a of the textile support 2, and configured to maintain the temperature of the user "U" constantly monitored, may also be provided.

In accordance with a first preferred embodiment, the sensor 21 is integrated inside the textile support 2 and positioned at the aforementioned hole (Figures 5a - 5c). In this way, the sensor 21 provides in a constant manner for the detection of the body temperature of the user "U", preferably a newborn.

According to a further embodiment not shown, instead, a sensor 21 is provided in the form of an external probe, positioned at the aforementioned hole, and attached to the skin of the user "U". In this case, the sensor 21 in the form of the probe may be removed from the support 2 without unbuckling the support 2 itself.

Furthermore, the item 1 comprises a transceiver 9 (Figures 1 to 4 and 6) of the wireless connection type and configured to receive the signals from the sensors 8 and forward them to a user interface device 10 which will be better described later.

The transceiver 9 has a substantially box-like conformation and is not described in detail as it is of a type known and widely used for the wireless reception and transmission of electromagnetic signals. This transceiver 9 can for example use known wireless transmission protocols such as for example Bluetooth^{®}, Wi-Fi, or other similar wireless communication systems.

The transceiver 9 internally comprises a battery 12 for the electric power supply of the sensors 8, 21 and all the electronic components, as well as an electronic unit for processing the signals received from at least one sensor 8, to send the respective electromagnetic signals to the interface device 10.

Advantageously, the transceiver 9 is engaged to the support 2 by means of a reversible hooking member 11 which allows to engage removably the transceiver 9, and then define a locked condition and a condition of quick release, to an outer surface 2b of the textile support 2 opposite to the inner surface 2a.

Preferably, in the condition of use of the support 2 which is closed in a ring (Figure 1), the hooking member 11 is configured to position the transceiver 9 externally and at the back of the user "U". In this context, the transceiver 9 does not cause any inconvenience in terms of comfort and wearability.

In fact, it should be noted that the transceiver 9 is positioned on an area of the newborn (the back) that always remains free in the prone position that the newborn assumes in the first hours of life and during lactation.

In particular, the reversible hooking member 11 comprises a first 11a and a second 11b element which can be engaged/disengaged from each other and respectively associated to the textile support 2 and to the transceiver 9.

In this context, the elements 11a, 11b of the hooking member 11 are arranged at the second end 3b of the band 3 and are switchable between a configuration of mechanical interlocking (Figures 1, 2 and 4) in which define the stable engagement of the transceiver 9 to the textile support 2, and a configuration of disengagement (Figure 3) wherein the transceiver 9 is removed from the textile item 2.

Preferably, the elements 11a, 11b are constituted by press-studs.

It should however be specified that the hooking member 11 can be of any type provided they are able to implement in a simple and manual manner actions of stable engagement and disengagement of the transceiver 9 from the support 2.

In accordance with a preferred embodiment of the invention, the first element 11a has a circular base 13, made of electrically conductive material and constrained in a stable manner to the outer surface 2b of the support 2. As is better illustrated in the enlargement of Figure 5b, from the base 13 and away from it extends a projecting pin 14 having an enlarged end portion 15 defining an interlocking undercut 15a.

Similarly, also the second element 11b presents a circular base 13, made of an electrically conductive material and constrained in a stable manner to a wall 9a of the transceiver 9. The second element 11b has also a hole 16 for housing the pin 14, which is formed at the centre of the base 13. Advantageously, in the engagement condition of the elements 11a, 11b, the respective bases 13 are approached by inserting the pin 14 into the hole 16 until the enlarged end 15 remains inserted inside the base 13. In this situation the interlocking undercut 15a is constrained within the hole 16 and in abutment with the edge that defines the hole 16 itself. Preferably, as is illustrated in the accompanying figures, are provided two first elements 11a and two respective second elements 11b aligned between them to be reciprocally coupled and for further support to the transceiver 9 to the textile support 2.

The item 1 also comprises electrical connection means 17 schematically shown in Figures 5a and 5b.

The connection means 17 are associated in the textile support 2 (and therefore not visible in their respective surfaces 2a and 2b) for transmitting the signals from each sensor 8, 21 and electrically transferring them to a respective first element 11a of the hooking member 11.

In other words, for each sensor 8, 21 and the respective first element 11a, an electrical connection through the means 17 extends, which runs along the longitudinal development of the band 3.

Advantageously, in accordance with a possible embodiment of the invention, the connection means 17 comprise electrically conductive elastic yarns, for example realized on a silver base, and having a first end 17a associated with the sensor 8 and a second end 17b opposite to the first end 17a and associated to the first element 11a.

In this case, the elastic yarns are conductive and a silver base or steel, are interwoven in the band 3 and extend parallel and in proximity of the longitudinal edges of the band 3.

Alternatively, the connection means can be constituted by a conductive multilayer print, integrated in the band 3.

In this case, materials such as nanosized graphene, particularly suitable in view of the intrinsic properties of elasticity, mechanical resistance and electrical conductivity can be used for printing the circuit.

The signal generated by each sensor 8 is then transmitted through the means 17 to a respective first element 11a which, being made of conductive material, in turn transmits the signal to the second element 11b.

The hooking member 11 therefore provides both the mechanical connection of the transceiver 9 to the support 2, and electrical connection between the means 17 and the transceiver 9.

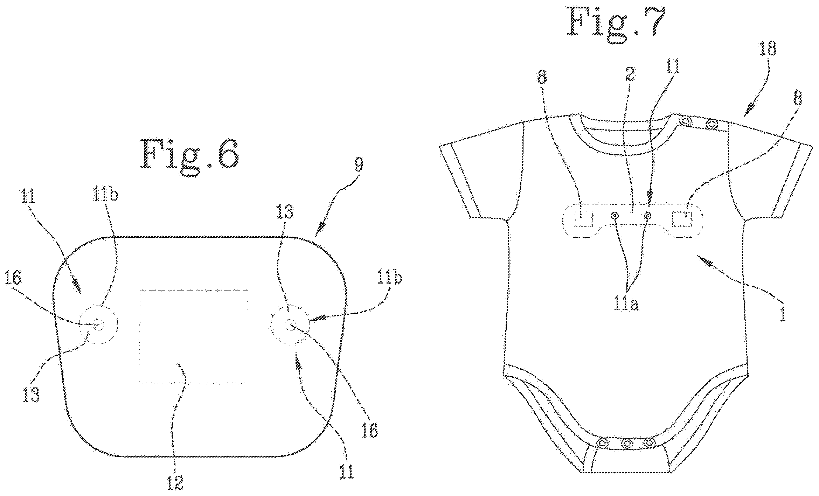

The sensorized textile item 1 described above can also be used for a wearable clothing item 18, such as for example a newborn bodysuit (figure 7) or another garment.

In this case, the clothing item 18 is configured to place the sensors 8 at the chest of the user "U".

In this embodiment, the textile item 1 is integrated into the clothing 18 and is always in the form of a band. However, in this embodiment the sensors 8 are arranged at the respective opposite ends.

Furthermore, in this situation the hooking member is interposed between the two sensors 8 and always externally, in order to place the transceiver 9 on the chest of the user.

This embodiment is in fact dedicated preferably for domestic use, where the newborn is mainly in the supine position, for the control of the physiological parameters during sleep and wake.

The present invention further relates to a system for monitoring the physiological parameters, in particular of a newborn, in the early hours of life, through the sensorized textile item 1 described above.

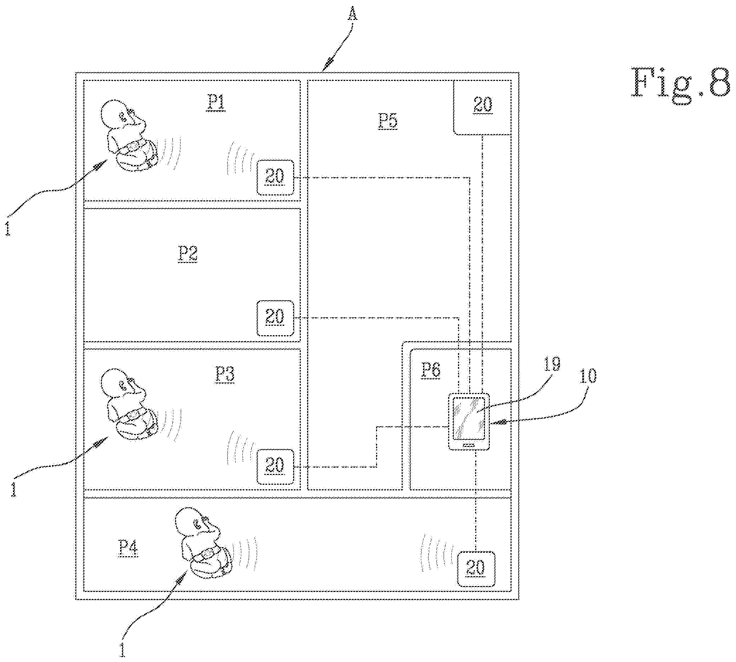

The system, which is illustrated in a schematic and exemplary manner in Figure 8, comprises the aforementioned user interface device 10, preferably of the mobile type such as for example a smartphone or a tablet. Alternatively, the user interface device 10 can also be constituted by a fixed-type computer, installed in a predefined control room.

The device 10 comprising a screen 19 (Figure 1) configured to display a graphic and/or textual indication of the signals representative of the physiological parameters of the newborn.

Advantageously, the system comprises a plurality of textile items 1, each of which can wirelessly send the signals to the device 10 and by means of the respective transceiver 9.

In this case, the screen 19 is configured to display the graphic/text indication representative of the physiological parameters of all newborns with which the textile items 1 are associated.

In accordance with a preferential embodiment of the present invention, which finds particular and advantageous application in environments such as for example the paediatric departments of hospitals, a plurality of gateway devices 20 are provided, interposed between the sensorized textile items 1 and the user interface device 10 for transferring the aforementioned signals.

In particular, each gateway device 20, consisting of an electronic network for the wireless transfer of data, is arranged in a respective portion P1 - P6 of an area "A" to be monitored.

In accordance with what is illustrated by way of example in Figure 8, the area "A" can be represented by the paediatric ward of a hospital that has to be monitored.

In this situation, the gateway devices 20 are arranged in each portion represented by patient rooms P1, P2, P3, from the delivery room P4, the department hallway P5 and a nurses station P6 (control room). Therefore, the entire area "A" is defined by the sum of the portions P1 - P6 inside which are housed the aforementioned gateway devices 20 adapted to detect the presence of sensorized textile items 1.

In fact, each gateway device 20 is configured to receive the signals from the respective transceivers 9 by means of a wireless communications protocol, such as in the particular the Bluetooth^{®} protocol.

Each gateway device 20 transmits the signals received from the item 1 to the interface device 10, by means of wireless transmission, for example by SmartLink system.

Therefore, the hospital professional located for example in the portion P6 of the area "A" receives all data relating to each item 1 from the gateway devices 20. These data are displayed in the single interface device 10 such as a tablet for a general and immediate control of the physiological conditions of each user "U" (newborn).

The presence of the aforementioned gateway devices 20 located in respective portions P1-P5 of area "A", also defines a geolocation device of each sensorized textile item 1, adapted to map the position of each item 1 inside a respective area "A".

The area "A" and the position of sensorized textile items 1, corresponding to the position of each user "U" in the portions P1-P5, is advantageously displayed on the screen 19.

Therefore, by using a single interface device 10 it is possible to maintain monitored the physiological activity of each user "U" and at the same time check any movements within the reference area.

The present invention further relates to a method for monitoring the physiological parameters, in particular of a newborn, in the early hours of life, through the sensorized textile item 1 described above.

The method is implemented by means of the above described system in mainly structural terms.

In particular, according to a first operating protocol used in a hospital setting, the method provides the steps of combining a code of the textile support 2 and a code of the transceiver 9 to the parturient in the registry of the electronic system of the department.

This combining step is carried out on the interface device 10 by means of a suitable application.

At the time of delivery, after cleaning the newborn, the textile support 2 is applied, wrapping the band 3 around the chest of the newborn as described above. The transceiver 9, with charged battery, is also applied to the textile support 2 and by means of the aforementioned reversible hooking member 11.

In this way, the monitoring via the interface device 10 is activated at the postpartum period ("skin to skin" period), in which the mother is in a predefined location (delivery room P4).

The signals received by the transceiver 9 are then transmitted from the same to the respective gateway device 20 via Bluetooth^{®}. In turn, the gateway device 20 resend data using SmartLink to the interface device 10. The data transmitted to the interface device 10 are:
- identification code of the newborn and possible description of reference;
- identification code of the mother;
- identification code of the sensorized textile item 1 coupled to the child;
- code of the gateway device 20 which detects the item 1;
- date and time.

In this way, it is possible to manage notifications or alarms to the interface device 10 and viewable by the hospital operator.

These notifications/alarms are representative of:
- the correct connection of the item 1 (online);
- the failed connection of the item 1 (offline);
- the incorrect position of the sensors 8;
- the electrocardiogram value;
- the breath of the newborn;
- the temperature of the newborn;
- the battery level of the transceiver 9.

With reference to the structure of the above described item 1, and in particular to the presence of the temperature sensor 21 is implemented a continuous detection of the temperature in order to maintain constantly monitored this parameter for each newborn.

In other words, the system allows a constant temperature control especially of the newborn in the early hours of life, which notoriously must remain within certain values. In fact, it is known that newborns can have temperature values too low with respect to normal physiological parameters. In this context, the temperature sensor 21 allows you to inform the medical staff about the temperature evolution and therefore draw this parameter steadily throughout the period of hospitalisation.

In addition, and as specified above, via the interface device 10 it is possible to check the position of each item 1 connected to the respective newborn. All gateway devices 20 map and thus provide the data relating to item 1 which enter the portion P1-P6 of the reference area "A". Advantageously, the system and method prove to be particularly useful precisely within the paediatric areas of hospitals, in order to give the staff an instrument suitable to constantly monitor all infants who wear the sensorized textile item 1.

Furthermore, the possibility of locating each newborn improves and optimizes the activity of the staff who can immediately trace and intervene on the newborn for any care or first aid activity.

In addition, the absence of a sensorized textile item 1 inside the reference area "A", such as for example the paediatric ward, allowing to detect any conditions of loss of the newborn with the consequent possibility of immediate intervention.

At the end of the hospital stay of the newborn, the item 1 is stopped and removed from the newborn, while the matching codes are freed.

The disposable textile support 2 is then discarded, while the transceiver 9 is dissociated from the support and put in energy charge for an advantageous new usage.

In accordance with a further operation protocol used in the domestic field, the method comprises the steps of combining the item 1 to the newborn, preferably by means of the clothing item 18 described above and which embeds the item 1.

In this situation, the gateway device 20 is optional since the transceiver 9 can communicate directly with the user interface device 10 managed by the parent. The gateway 20 can be used for remote data transfer and possibly equipped with a microphone for voice transmission to the interface device 10.

A special application is also used for monitoring the newborn both at home and outside home.

In this way, it is possible to manage notifications or alarms to the interface device 10 and viewable by parents.

These notifications/alarms are representative of:
- the correct connection of the item 1 (online);
- the failed connection of the item 1 (offline);
- the incorrect position of the sensors 8;
- the electrocardiogram value;
- the breath of the newborn;
- the temperature of the newborn;
- the position of the newborn;
- the movement of the newborn;
- the battery level of the transceiver 9.

The above described invention solves the drawbacks found in the prior art and provides significant advantages.

In the first place, the item 1 is particularly comfortable due to the absence of rigid components inside the support 2 which are potentially harmful to the user.

This advantage is given precisely by the possibility of reversibly coupling the transceiver 9, the only component that can be made with rigid materials and large volumes, outside the support 2. In this way, the transceiver 9 never comes into contact with the user.

Therefore, even due to excessive pressure of the band 3 around the body of the user "U", uncomfortable situations in which rigid parts of the item 1 press on the body are not configured.

In addition, again thanks to the external reversible connection of the transceiver 9, the entire support 2 is entirely breathable as it is made of fabric. In this regard, it should also be considered that only a small part of the torso of the user "U" is wrapped by band 3, significantly limiting the area affected by the presence of the sensorized textile item 1.

Even the electrical contact parts made of non-breathable material, are in any case limited significantly to the mere presence of the two first elements 11a made of conductive material.

Advantageously, the skin of the user is protected from possible damage and always kept in the optimal humidity conditions.

A further important advantage always derived from the possibility of removing the transceiver 9 from the support 2, is given by the possibility of keeping the support 2 constantly worn.

The operations of replacing the sensorized textile item 1 are in fact significantly limited, since the transceiver which includes the battery 12 can be removed for electrical charge and all the care interventions on the newborn can be implemented with the item 1.

The support 2 in the form of a band 3 wrapped around the torso is also preserved from any physiological liquid precisely because it is positioned in an area of the body far from the infant's mouth.

Furthermore, the item 1 proves to be very versatile and can be used for any size of the user "U".

As described above, this advantage is given by the fact that the textile item 1 is incorporated in a single band 3 and by the presence of the engagement means 4 which may be adjustable in function of the size of the user "U".

Therefore, it is possible to use a single textile support 2 for different types of users, with the consequent advantages in economic terms and ease of use.

Another important advantage of the present invention is given by the structural simplicity of the entire sensorized item 1.

In fact, the presence of a textile support 2 in the form of a simple band 3 allows to simplify the positioning operations of the item 1 always maintaining a precise position of the sensors 8 with respect to the user "U".

Even the operations for producing the entire item 1 are simplified since all the electronic components are sewn and/or printed to a support 2 in the form of a simple fabric band.

Finally, the system and the method thereof, which use the item 1, turn out to be particularly advantageous in terms of practicality of use and versatile as they can be used both in a hospital context, for monitoring the physiological parameters and the position of a plurality of newborns wearing the item 1, and in a home environment for controlling a respective newborn.

The possibility of disconnecting the transceiver 9, and therefore the electronic components dedicated to the power supply, data processing and transmission, also makes the system extremely economical and usable for a series of users "U". In this case it should be noted that only the textile support 2 is disposable while the transceiver 9 can be associated and reused over time with different textile items 1.

## Claims

1. Sensorized textile item (1) for the detection and transmission of physiological parameters, including:
- a textile support (2), made of elastic material and configured to be worn by a newborn (U); said support (2) comprising a band (3) having a rectangular peripheral development and defining respective opposite ends (3a, 3b);
- at least one sensor (8) arranged at an inner surface (2a) of the textile support (2) to be in contact with the newborn (U), said sensor (8) being configured to detect a plurality of signals representative of respective physiological parameters of the newborn (U); and
- a wireless transceiver (9) configured to receive said signals from the sensor (8) and forward them to a user interface device (10);
- a reversible hooking member (11) of said transceiver (9) to an outer surface (2b) of said textile support (2) opposite said inner surface (2a); said hooking member (11) presenting a first and a second element (11a, 11b) engageable/disengageable from each other and respectively associated to the textile support (2) and to the transceiver (9); **characterized in that** said support (2) further comprising resolvable engagement means (4) arranged at said ends (3a, 3b) to close the band (3) in a ring around the newborn (U), the sensor (8) being arranged proximate to a first end (3a) of said ends (3a, 3b) of the band (3), the first and the second element (11a, 11b) of the reversible hooking member (11) being arranged at a second end (3b) of said ends (3a, 3b) of the band (3), for arranging said at least one sensor (8) at the chest of the newborn (U), and said reversible hooking member (11) at the back of the newborn (U), and said resolvable engagement means (4) at the side of the newborn (U).

2. Item according to the previous claim, **characterized in that** it also comprises electrical connection means (17) associated in said textile support (2) for transmitting said signals from the sensor (8) and electrically transferring them to said first element (11a).

3. Item according to any one of the preceding claims, **characterized in that** said at least one sensor (8) comprises a contact surface (8a) in contact with the skin of the newborn (U) emerging from said inner surface (2a) and protruding from it.

4. Item according to claim 2 or 3, **characterized in that** said connection means (17) comprise electrically conductive elastic yarns and having a first end (17a) associated with said at least one sensor (8) and a second end (17b) opposite to first (17a) and associated with said first element (1 1a).

5. Item according to any one of the preceding claims, **characterized in that** said elements (11a, 11b) of the reversible hooking member (11) are switchable between a mechanical interlocking configuration in which they define the stable engagement of the transceiver (9) to the textile support (2), and a disengagement configuration wherein the transceiver (9) is removed from the textile support (2).

6. Item according to the previous claim, **characterized in that** the first element (11a) comprises a protruding pin (14) having an enlarged end portion (15) defining an interlocking undercut (15a); and **in that** said second element (11b) has a hole (16) for housing said protruding pin (14); said interlocking undercut (15a) being constrained inside said hole (16) in the respective interlocking configuration.

7. Item according to any of the foregoing claims, **characterized in that** said transceiver (9) comprises an electric power supply battery (12) and an electronic signal processing unit received from at least one sensor (8), to send respective electromagnetic signals.

8. Item according to claim 1, **characterized in that** said resolvable engagement means (4) comprise at least one through opening (5) made in a first end (3a) of the band (3) and at least one strap (6) integrally made with said band (3) and protruding from a second end (3b) of the band (3) itself; said strap (6) being insertable in the through opening (5) for reversibly engaging the ends (3a, 3b) between each other.

9. Monitoring system of physiological parameters, in particular of a newborn, **characterized in that** it comprises a textile item (1) according to any one of claims from 1 to 8, and an user interface device (10) of the mobile type having a display (19) configured to display a graphic and/or text indication of the signals representative of the physiological parameters of the newborn.

10. System according to the previous claim, **characterized in that** it comprises a plurality of sensorized textile items (1); said display (19) being configured to display the graphic/text indication representative of the physiological parameters of all newborns to which said textile items are associated (1).

11. System according to any one of claims 9 or 10, **characterized in that** it further comprises a plurality of gateway devices (20), interposed between the sensorized textile items (1) and the user interface device (10) for transferring said signals.

12. System according to the previous claim, **characterized in that** each gateway device (20) is arranged in a respective portion (P1 - P5) of an area (A) to be monitored, to detect the presence of one or more sensorized textile items (1) inside the respective portion (P1 - P5).

13. System according to claim 11 or 12, **characterized in that** each gateway device (20) receives signals from the transceivers (9) of respective items (1) by means of the Bluetooth^{®} communication protocol, and **in that** it transmits said signals to the interface device (10) through a SmartLink system.

14. Method for monitoring physiological parameters, in particular of a newborn, comprising the steps of:
- wearing a textile support (2) of a sensorized textile item (1) according to any one of claims 1 to 8, by a respective newborn (U);
- engaging the transceiver (9) to the textile support (2) by means of the reversible hooking member (11);
- activating the communication between the transceiver (9) and the user interface device (10); and
- displaying on a screen (19) of the interface device (10) a graphic and/or text indication of the signals representative of the physiological parameters of said newborn (U); **characterized in that**, prior to the step of wearing the textile support (2), the step of matching, into said interface device (10), a code of the textile support (2) and a transceiver code (9) to a mother; said support (2) being worn by the newborn of said mother, is implemented; and **in that** said step of activating the communication between the transceiver (9) and the user interface device (10) is carried out by means of one or more gateway devices (20) arranged in correspondence a respective portion (P1-P6) of an area (A) to be monitored; said transceiver (9) sending data to the gateway device (20) via Bluetooth^{®}; said gateway device (20) forwarding the data to the interface device (10).

15. Method according to the previous claim, **characterized in that** said data transmitted to the interface device (10) determine respective notifications that can be displayed on the screen (19) and are representative:
- of the correct connection of the item 1 (online); and/or
- of the failed connection of the item 1 (offline); and/or
- of the incorrect position of the sensors (8); and/or
- of the electrocardiogram value; and/or
- of the breath of the newborn; and/or
- of the temperature of the newborn; and/or
- of the battery level 12 of the transceiver (9) and/or
- of the position of the item 1 within the area (A).
